# FASCICULE DE BREVET EUROPEEN

(11) **EP 0 898 959 B1**
(45) Date de publication et mention de la délivrance du brevet: **19.12.2001**
(21) Numéro de dépôt: 98401987.7
(22) Date de dépôt: 04.08.1998
(51) Int. Cl.: A61K 7/48, A61K 7/02, A61K 7/047

(54) **Composition démaquillante**
Abschminkmittel
Make-up removing composition

(30) Priorité: 28.08.1997 FR 9710757
(43) Date de publication de la demande: 03.03.1999
(73) Titulaire: L'OREAL, 75008 Paris (FR)
(72) Inventeur: Ramin, Roland, 75014 Paris (FR); Arnaud, Pascal, 94340 L'Hay-Les-Roses (FR)
(74) Mandataire: Lhoste, Catherine

(56) Documents cités:
- EP-A- 0 651 990
- EP-A- 0 776 655
- MAJEWICZ: "oil-based cosmetic and therapeutic compositions containing ethylguar" RESEARCH DISCLOSURE, no. 37807, octobre 1995, page 642 XP002067846
- CLARKE ET AL.: "Ethyl galactomannan film properties for use in personal care applications" RESEARCH DISCLOSURE, no. 38413, avril 1996, pages 235-236, XP002067847

## Description

La présente invention se rapporte à des compositions de dissolvant de vernis à ongles ainsi qu'à l'utilisation pour la préparation d'une composition et/ou dans une composition de dissolvant de vernis à ongles, d'un alkyléther de polysaccharide associé à un système démaquillant.

Les compositions de démaquillage des matières kératiniques classiquement utilisées, se présentent généralement sous forme de lotion ou de lait. En outre, elles contiennent des huiles et/ou des tensioactifs permettant l'élimination du maquillage. Une telle composition est notamment décrite dans les demandes EP-A-705592, EP-A-651990 et EP-A-776655.

Ces compositions connues, bien que très efficaces, présentent un certains nombres d'inconvénients. En particulier, elles sont souvent difficiles à prélever et peuvent s'écouler entre les doigts.

Pour épaissir les compositions, il est connu d'ajouter un agent épaississant dans la dite composition. Lorsque la composition comprend majoritairement une phase non aqueuse, c'est-à-dire comprenant soit un solvant organique, soit une huile, on utilise de manière connue un épaississant de nature lipophile. Ainsi, on peut par exemple utiliser une argile comme la bentonite pour épaissir une phase grasse mais cette argile à l'inconvénient de rendre opaque la composition épaissie.

Il est également connu d'utiliser de la silice pyrogénée pour épaissir une phase non aqueuse, mais les compositions ainsi épaissies ont tendance à être translucides.

En outre, ces épaississants de nature lipophile ne sont compatibles qu'avec un nombre limité de solvants et d'huiles couramment utilisés dans les démaquillants, limitant ainsi la variété des formulations de ces compositions.

Un besoin subsiste de disposer d'un démaquillant sous forme gélifié plus ou moins fluide présentant à la fois des propriétés de transparence et une bonne rhéologie.

Le demandeur a constaté que ce but pouvait être atteint en utilisant, à titre d'épaississant, un alkyléther de polysaccharide particulier dans une composition démaquillante. Cet épaississant permet de préparer des compositions démaquillantes se présentant sous forme gélifiée pouvant être transparente. Ces compositions gélifiées présentent par ailleurs une bonne rhéologie pouvant aller d'une texture fluide à une texture épaisse, selon le souhait du formulateur.

En outre, cet épaississant est compatible avec de nombreux solvants et huiles, permettant ainsi d'utiliser une grande variété de solvants et d'huiles dans les compositions démaquillantes et d'envisager de nouveaux produits cosmétiques.

L'invention a donc pour objet l'utilisation pour la préparation d'une composition et/ou dans une composition de dissolvant de vernis à ongles d'un alkyléther de polysaccharide formé de motifs comportant au moins deux cycles osidiques différents, chaque motif comportant au moins un groupe hydroxyle substitué par une chaîne alkyle hydrocarbonée saturée associé à un système démaquillant comprenant au moins un solvant non aqueux.

Dans l'épaississant de l'invention, on entend par « chaîne alkyle hydrocarbonée » une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment méthyle, éthyle, éthényle, n-propyle, propényle, isopropyle, n-butyle, isobutyle, tertiobutyle, n-pentyle. Ces alkyléthers peuvent être fabriqués comme décrits dans les documents EP-A-281 360, EP-A-708 114, EP-A-281360.

Selon un mode de réalisation préféré de l'invention, l'alkyléther de polysaccharide a un poids moléculaire moyen en poids supérieur à 100 000, et de préférence supérieur à 200 000. Ce poids moléculaire peut aller jusqu'à 1 million. Cet alkyléther peut comporter de un à six et mieux de deux à quatre groupes hydroxyle par motif, substitués par une chaîne alkyle hydrocarbonée saturée ou non.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique. Comme gommes appropriées, on peut citer par exemple la gomme de guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme de guar. Ainsi, avantageusement l'alkyéther est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,8, tel que décrit dans les documents RD 95378007 (octobre 1995) et EP-A-708114. Cette gomme est en particulier celle vendue par la société Aqualon sous les noms N-HANCE-AG 200® et N-HANCE AG 50® .

La concentration en alkyléther dépend de la forme galénique, de la consistance recherchées pour la composition démaquillante. En particulier le rapport en poids de la quantité de solvant et/ou d'huile sur la quantité d'épaississant est choisi par exemple dans la gamme allant de 5 à 1000. La composition selon l'invention peut contenir par exemple une quantité d'alkyléther de polysaccharide allant de 0,1 à 20 % du poids total de la composition, et de préférence de 2 % à 8 % en poids, et mieux de 3 % à 6 % en poids.

Le système démaquillant peut comprendre au moins un solvant organique. Celui-ci peut être notamment choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde ;

On utilise plus préférentiellement les esters et les cétones ayant au plus 10 atomes de carbone et plus particulièrement la méthyléthylcétone, l'acétate d'éthyle, l'acétate de méthyle ou l'acétate de butyle.
Les solvants peuvent être présents à raison de 0 à 99,9 % en poids, par rapport au poids total de la composition, et mieux de 20 % à 98 % en poids.

Aussi, l'invention a encore pour objet un dissolvant de vernis à ongles contenant au moins un solvant choisi parmi la méthylisobutylcétone, la diisobutylcétone, l'isophorone, la cyclohexanone, l'isopropanol, le diacétone alcool, le 2-butoxyéthanol, le cyclohexanol, l'acétate de méthyle, l'acétate de propyle, l'acétate de n-butyle, l'acétate d'isopentyle, le diéthyléther, le diméthyléther, le dichlorodiéthyléther, le décane, le dodécane, le cyclohexane, le xylène, le benzaldéhyde, l'acétaldéhyde.

La composition gélifiée résultant de l'association de l'alkyléther de polysaccharide et du système démaquillant tels que définis précédemment peut être utilisée tel quelle et constituer elle-même une composition pour le démaquillage des matières kératiniques. Elle peut également être incorporée dans une formulation plus complexe de démaquillage des matières kératiniques en une quantité efficace pour obtenir à la fois la texture et la viscosité souhaitées et un bon démaquillage des matières kératiniques.

En outre, on peut ajouter de préférence une phase aqueuse dans une quantité allant de 0,1 à 30 % en poids par rapport au poids total de la composition, et mieux de 5 % à 10 % en poids.

Elles peuvent contenir en plus des adjuvants classiques tels que des colorants, des parfums, des conservateurs, des filtres solaires, des agents hydratants. Ces adjuvants sont présents dans des quantités allant de préférence de 0 à 20% en poids par rapport au poids de la composition.

L'invention concerne également un procédé de démaquillage des ongles, caractérisé par le fait qu'on applique sur la surface de la matière kératinique une quantité efficace d'une composition constituée par ou contenant un gel formé à partir d'un alkyléther de polysaccharide et d'un système démaquillant comprenant un solvant non aqueux.

Les exemples ci-après sont donnés à titre d'illustration et sans caractère limitatif.

### Exemple 1 :

On a préparé une lotion démaquillante de vernis à ongles ayant la composition suivante :
- acétate d'éthyle 77,5 g
- guar éthylé de degré de substitution d'environ 2,5 (1) 4 g
- glycérine 2 g
- alcool éthylique 12 g
- eau 4 g
(1) vendu sous la dénomination N-HANCE AG 200® par Aqualon

On obtient une lotion transparente qui démaquille facilement un film de vernis à ongles.

### Exemple 2 :

On a préparé une lotion démaquillante transparente ayant la composition suivante
- acétone 83,5 g
- alcool éthylique 11g
- guar éthylé de degré de substitution d'environ 2,5 (1) 4 g
- glycérine 2 g
(1) vendu sous la dénomination N-HANCE AG 200® par Aqualon

Cette lotion fluide, légèrement épaissie, convient pour le démaquillage des ongles.

## Revendications

1. Utilisation pour la préparation d'une composition et/ou dans une composition de dissolvant de vernis à ongles, d'un alkyléther de polysaccharide formé de motifs comportant au moins deux cycles osidiques différents, chaque motif comportant au moins un groupe hydroxyle substitué par une chaîne alkyle hydrocarbonée saturée associé à un système démaquillant comprenant au moins un solvant non aqueux.

2. Utilisation selon la revendication 1, **caractérisée par le fait que** deux à quatre groupes hydroxyle par motif sont substitués par une chaîne alkyle hydrocarbonée saturée.

3. Utilisation selon l'une quelconque des revendications précédentes, **caractérisée par le fait que** la chaîne alkyle hydrocarbonée saturée comporte de 1 à 24 atomes de carbone.

4. Utilisation selon l'une quelconque des revendications précédentes, **caractérisée par le fait que** la chaîne alkyle hydrocarbonée saturée comporte de 2 à 10 atomes de carbone.

5. Utilisation selon l'une quelconque des revendications précédentes, **caractérisée par le fait que** la chaîne alkyle est choisie dans le groupe formé par les radicaux méthyle, éthyle, n-propyle, isopropyle, n-butyle, isobutyle, tertiobutyle.

6. Utilisation selon l'une quelconque des revendications précédentes, **caractérisée par le fait que** les cycles osidiques sont choisis dans le groupe formé par le mannose, le galactose, le glucose, le furanose, le rhamnose, l'arabinose.

7. Utilisation selon l'une quelconque des revendications précédentes, **caractérisée par le fait que** l'alkyléther de polysaccharide est un alkyléther d'une gomme choisie parmi la gomme guar, la gomme de caroube, la gomme de karaya, la gomme adragante et leurs mélanges.

8. Utilisation selon l'une quelconque des revendications précédentes, **caractérisée par le fait que** l'alkyéther est un galactomannane alkylé de chaîne alkyle en C₁ à C₆ et mieux en C₁ à C₃.

9. Utilisation selon l'une quelconque des revendications précédentes, **caractérisée par le fait que** l'alkyléther de polysaccharide est de la gomme de guar à chaîne éthyle avec un degré de substitution de 2 à 3.

10. Utilisation selon l'une quelconque des revendications précédentes, **caractérisée par le fait que** l'alkyléther de polysaccharide a un poids moléculaire moyen en poids supérieur à 200 000.

11. Utilisation selon l'une quelconque des revendications précédentes, **caractérisée par le fait que** l'alkyléther de polysaccharide est présent en une quantité telle que le rapport (en poids) de la quantité de solvant et/ou d'huile sur la quantité d'épaississant est choisi dans la gamme allant de 5 à 1000.

12. Utilisation selon l'une quelconque des revendications précédentes, **caractérisée par le fait que** l'alkyléther de polysaccharide est présent en une quantité allant de 0,1 à 20 % du poids total de la composition et mieux de 2 à 8 % du poids total de la composition.

13. Utilisation selon l'une quelconque des revendications précédentes, **caractéri-sée par** le fait le solvant non aqueux est choisi dans le groupe formé par les cétones, les alcools, les glycols, les esters à chaîne courte ayant de 3 à 8 atomes de carbone, les éthers, les alcanes, les composés cycliques aromatiques, les aldéhydes liquides à température ambiante.

14. Utilisation selon l'une quelconque des revendications précédentes, **caractérisée par le fait que** le solvant non aqueux est choisi dans le groupe formé par la méthyléthylcétone, l'acétate d'éthyle, l'acétate de méthyle, l'acétate de propyle ou l'acétate de butyle.

15. Procédé de démaquillage des ongles, **caractérisée par le fait qu'**on applique sur la surface de la matière kératinique une quantité efficace d'une composition constituée par ou contenant un gel formé à partir d'un alkyléther de polysaccharide selon la revendication 1 et et d'un système démaquillant comprenant un solvant non aqueux.

16. Dissolvant de vernis à ongles comprenant au moins un alkyléther de polysaccharide formé de motifs comportant au moins deux cycles osidiques différents, chaque motif comportant au moins un groupe hydroxyle substitué par une chaîne alkyle hydrocarbonée saturée,
et au moins un solvant choisi parmi la méthylisobutylcétone, la diisobutylcétone, l'isophorone, la cyclohexanone, l'alcool isopropylique, le diacétone alcool, le 2-butoxyéthanol, le cyclohexanol, l'acétate de méthyle, l'acétate de n-butyle, l'acétate d'isopentyle, le diéthyléther, le diméthyléther, le dichlorodiéthyléther, le décane, le dodécane, le cyclohexane, le xylène, le benzaldéhyde, l'acétaldéhyde.

17. Dissolvant selon la revendication 16, **caractérisé par le fait que** l'alkyléther de polysaccharide est une gomme de guar éthylée ayant un degré de substitution de 2 à 3.

## Patentansprüche

1. Verwendung eines Polysaccharidalkylethers, der aus Einheiten gebildet ist, die mindestens zwei verschiedene Zuckerringe aufweisen, wobei jede Einheit mindestens eine Hydroxygruppe enthält, die mit einer gesättigten Alkylgruppe (Kohlenwasserstoffgruppe) substituiert ist, in Kombination mit einem Abschminksystem, das mindestens ein nicht wäßriges Lösungsmittel enthält, zur Herstellung von Zusammensetzungen und/oder in Zusammensetzungen, die einen Nagellack auflösen können.

2. Verwendung nach Anspruch 1, **dadurch gekennzeichnet, daß** pro Einheit 2 bis 4 Hydroxygruppen mit einer gesättigten Alkylgruppe substituiert sind.

3. Verwendung nach einem der vorhergehenden Ansprüche, **dadurch gekennzeichnet, daß** die gesättigte Alkylgruppe 1 bis 24 Kohlenstoffatome aufweist.

4. Verwendung nach einem der vorhergehenden Ansprüche, **dadurch gekennzeichnet, daß** die gesättigte Alkylgruppe 2 bis 10 Kohlenstoffatome aufweist.

5. Verwendung nach einem der vorhergehenden Ansprüche, **dadurch gekennzeichnet, daß** die Alkylgruppe unter Methyl, Ethyl, n-Propyl, Isopropyl, n-Butyl, Isobutyl und tert.-Butyl ausgewählt ist.

6. Verwendung nach einem der vorhergehenden Ansprüche, **dadurch gekennzeichnet, daß** die Zuckerringe unter Mannose, Galactose, Glucose, Furanose, Rhamnose und Arabinose ausgewählt sind.

7. Verwendung nach einem der vorhergehenden Ansprüche, **dadurch gekennzeichnet, daß** der Polysaccharidalkylether ein Alkylether eines Gummis ist, das unter Guargummi, Johannesbrotkernmehl, Karaya-Gummi und Tragant und deren Gemischen ausgewählt ist.

8. Verwendung nach einem der vorhergehenden Ansprüche, **dadurch gekennzeichnet, daß** es sich bei dem Alkylether um ein alkyliertes Galactomannan mit einer C₁₋₆-Alkylgruppe und besser noch einer C₁₋₃-Alkylgruppe handelt.

9. Verwendung nach einem der vorhergehenden Ansprüche, **dadurch gekennzeichnet, daß** der Polysaccharidalkylether ein Guargummi mit Ethylgruppe ist, das einen Substitutionsgrad von 2 bis 3 aufweist.

10. Verwendung nach einem der vorhergehenden Ansprüche, **dadurch gekennzeichnet, daß** der Polysaccharidalkylether ein Gewichtsmittel des Molekulargewichts über 200 000 aufweist.

11. Verwendung nach einem der vorhergehenden Ansprüche, **dadurch gekennzeichnet, daß** der Polysaccharidalkylether in einem solchen Mengenanteil vorliegt, daß das Verhältnis (auf das Gewicht bezogen) der Menge des Lösungsmittel und/oder Öls und der Menge des Verdickungsmittels im Bereich von 5 bis 1000 liegt.

12. Verwendung nach einem der vorhergehenden Ansprüche, **dadurch gekennzeichnet, daß** der Polysaccharidalkylether in einem Mengenanteil von 0,1 bis 20 % des Gesamtgewichts der Zusammensetzung und besser noch 2 bis 8 % des Gesamtgewichts der Zusammensetzung vorliegt.

13. Verwendung nach einem der vorhergehenden Ansprüche, **dadurch gekennzeichnet, daß** das nicht wäßrige Lösungsmittel unter den bei Raumtemperatur flüssigen Ketonen, Alkoholen, Glykolen, kurzkettigen Estern mit 3 bis 8 Kohlenstoffatomen, Ethern, Alkanen, cyclischen aromatischen Verbindungen und Aldehyden ausgewählt ist.

14. Verwendung nach einem der vorhergehenden Ansprüche, **dadurch gekennzeichnet, daß** das nicht wäßrige Lösungsmittel Methylethylketon, Ethylacetat, Methylacetat, Propylacetat oder Butylacetat ausgewählt ist.

15. Verfahren zum Abschminken der Nägel, **dadurch gekennzeichnet, daß** auf die Oberfläche der Keratinsubstanzen eine wirksame Menge einer Zusammensetzung aufgetragen wird, die aus einem Gel besteht oder ein Gel enthält, das aus einem Polysaccharidalkylether nach Anspruch lund einem Abschminksystem, das ein nicht wäßriges Lösungsmittel enthält, gebildet ist.

16. Nagellackentfemer, der mindestens einen Polysaccharidalkylether, der aus Einheiten gebildet wird, die mindestens zwei verschiedene Zuckerringe aufweisen, wobei jede Einheit mindestens eine Hydroxygruppe enthält, die mit einer gesättigten Alkylgruppe (Kohlenwasserstoffgruppe) substituiert ist, und mindestens ein Lösungsmittel enthält, das unter Methylisobutylketon, Diisobutylketon, Isophoron, Cyclohexanon, Isopropylalkohol, Diacetonalkohol, 2-Butoxyethanol, Cyclohexanol, Methylacetat, n-Butylacetat, Isopentylacetat, Diethylether, Dimethylether, Dichlordiethylether, Decan, Dodecan, Cyclohexan, Xylol, Benzaldehyd und Acetaldehyd ausgewählt ist.

17. Nagellackentferner nach Anspruch 16, **dadurch gekennzeichnet, daß** der Polysaccharidalkylether ein Ethylguargummi ist, das einen Substitutionsgrad von 2 bis 3 aufweist.

## Claims

1. Use, for the preparation of a composition and/or in a nail-varnish-dissolver composition, of a polysaccharide alkyl ether formed of units containing at least two different saccharide rings, each unit containing at least one hydroxyl group substituted with a saturated hydrocarbon-based alkyl chain combined with a make-up-removing system comprising at least one non-aqueous solvent.

2. Use according to Claim 1, **characterized in that** two to four hydroxyl groups per unit are substituted with a saturated hydrocarbon-based alkyl chain.

3. Use according to either of the preceding claims, **characterized in that** the saturated hydrocarbon-based alkyl chain contains from 1 to 24 carbon atoms.

4. Use according to any one of the preceding claims, **characterized in that** the saturated hydrocarbon-based alkyl chain contains from 2 to 10 carbon atoms.

5. Use according to any one of the preceding claims, **characterized in that** the alkyl chain is chosen from the group formed by methyl, ethyl, n-propyl, isopropyl, n-butyl, isobutyl and tert-butyl radicals.

6. Use according to any one of the preceding claims, **characterized in that** the saccharide rings are chosen from the group formed by mannose, galactose, glucose, furanose, rhamnose and arabinose.

7. Use according to any one of the preceding claims, **characterized in that** the polysaccharide alkyl ether is an alkyl ether of a gum chosen from guar gum, carob gum, karaya gum and gum tragacanth, and mixtures thereof.

8. Use according to any one of the preceding claims, **characterized in that** the alkyl ether is an alkyl galactomannan with a C₁ to C₆, and better still C₁ to C₃, alkyl chain.

9. Use according to any one of the preceding claims, **characterized in that** the polysaccharide alkyl ether is guar gum containing an ethyl chain with a degree of substitution of from 2 to 3.

10. Use according to any one of the preceding claims, **characterized in that** the polysaccharide alkyl ether has a weight-average molecular weight of greater than 200,000.

11. Use according to any one of the preceding claims, **characterized in that** the polysaccharide alkyl ether is present in an amount such that the ratio (by weight) of the amount of solvent and/or of oil to the amount of thickener is chosen in the range from 5 to 1000.

12. Use according to any one of the preceding claims, **characterized in that** the polysaccharide alkyl ether is present in an amount ranging from 0.1 to 20% of the total weight of the composition, and better still from 2 to 8% of the total weight of the composition.

13. Use according to any one of the preceding claims, **characterized in that** the non-aqueous solvent is chosen from the group formed by ketones, alcohols, glycols, short-chain esters containing from 3 to 8 carbon atoms, ethers, alkanes, cyclic aromatic compounds and aldehydes that are liquid at room temperature.

14. Use according to any one of the preceding claims, **characterized in that** the non-aqueous solvent is chosen from the group formed by methyl ethyl ketone, ethyl acetate, methyl acetate, propyl acetate or butyl acetate.

15. Process for removing varnish from nails, **characterized in that** an effective amount of a composition consisting of or containing a gel formed from a polysaccharide alkyl ether according to Claim 1 and from a varnish-removing system comprising a non-aqueous solvent, is applied to the surface of the keratinous material.

16. Nail varnish dissolver comprising at least one polysaccharide alkyl ether formed of units containing at least two different saccharide rings, each unit containing at least one hydroxyl group substituted with a saturated hydrocarbon-based alkyl chain, and at least one solvent chosen from methyl isobutyl ketone, diisobutyl ketone, isophorone, cyclohexanone, isopropyl alcohol, diacetone alcohol, 2-butoxyethanol, cyclohexanol, methyl acetate, n-butyl acetate, isopentyl acetate, diethyl ether, dimethyl ether, dichlorodiethyl ether, decane, dodecane, cyclohexane, xylene, benzaldehyde and acetaldehyde.

17. Dissolver according to Claim 16, **characterized in that** the polysaccharide alkyl ether is an ethyl guar gum having a degree of substitution of from 2 to 3.
